# FASCICULE DE BREVET EUROPEEN

(11) **EP 1 056 698 B1**
(45) Date de publication et mention de la délivrance du brevet: **10.12.2003**
(21) Numéro de dépôt: 99905981.9
(22) Date de dépôt: 25.02.1999
(51) Int. Cl.: C07C 17/20, C07C 17/21, C07C 19/08, C07C 17/00, C07C 21/18, C07C 19/10

(54) **PROCEDE D'HYDROFLUORATION D'HYDROCARBURES CHLORES**
VERFAHREN ZUR HYDROFLUORIERUNG VON CHLORIERTEN KOHLENWASSERSTOFFEN
HYDROFLUORINATION OF CHLORINATED HYDROCARBONS

(30) Priorité: 26.02.1998 BE 9800150
(43) Date de publication de la demande: 06.12.2000
(73) Titulaire: SOLVAY (Société Anonyme), 1050 Bruxelles (BE)
(72) Inventeur: WILMET, Vincent, B-1301 Wavre (BE); JANSSENS, Francine, B-1800 Vilvoorde (BE)
(74) Mandataire: Jacques, Philippe
(86) Numéro de dépôt international: BE9900028
(87) Numéro de publication internationale: WO99043635

(56) Documents cités:
- EP-A- 0 703 205
- EP-A- 0 712 826
- WO-A-95/27688
- WO-A-96/40605
- GB-A- 2 313 118
- DATABASE WPI Section Ch, Week 9826 Derwent Publications Ltd., London, GB; Class A60, AN 98-292060 XP002105127 & JP 10 101593 A (ASAHI GLASS CO LTD) , 21 avril 1998

## Description

La présente invention concerne un procédé d'hydrofluoration d'un hydrocarbure chloré par réaction avec du fluorure d'hydrogène en présence d'un catalyseur d'hydrofluoration, et en particulier un procédé de fabrication du 1,1,1,3,3-pentafluoroprapane.

Des procédés d'hydrofluoration, en phase liquide, basés sur la réaction du fluorure d'hydrogène et d'un composé organique chloré, en présence d'un catalyseur, sont connus depuis longtemps. Les produits recherchés sont des composés organiques analogues au composé organique chloré engagé dans lequel les atomes de chlore ont été partiellement ou totalement remplacés par des atomes de fluor. Cependant, le taux de transformation des réactifs engagés est souvent faible et la sélectivité en produit désiré insuffisante, en particulier lorsqu'une fluoration complète est souhaitée. Dans certains cas, plusieurs étapes sont nécessaires pour obtenir les produits fluorés recherchés. Ainsi, la demande de brevet WO 97/24307 décrit la synthèse du 1,1,1,3,3-pentafluoropropane (HFC-245fa) au départ de 1,1,1,3,3-pentachloroprapane en 2 étapes. Le 1,1,1,3,3-pentachloropropane réagit d'abord en phase gazeuse avec du fluorure d'hydrogène pour donner du 1,1,1-trifluoro-3-chloro-2-propène qui, après élimination du chlorure d'hydrogène formé, réagit dans une seconde étape avec du fluorure d'hydrogène pour donner le HFC-245fa.

Il est dès lors intéressant de disposer d'un procédé d'hydrofluoration performant permettant de remplacer plus aisément et avec une grande sélectivité des atomes de chlore par des atomes de fluor.

Par hydrofluoration, on entend la réaction d'addition de fluorure d'hydrogène sur une double liaison carbone-carbone ainsi que la réaction de substitution d'un atome de chlore par un atome de fluor sur un substrat saturé.

L'invention concerne dès lors un procédé d'hydrofluoration d'un hydrocarbure chloré par réaction avec du fluorure d'hydrogène, dans un milieu réactionnel comprenant un catalyseur d'hydrofluoration, qui se caractérise par une alimentation continue de chlorure d'hydrogène dans le milieu réactionnel.

Dans le procédé d'hydrofluoration selon la présente invention, on entend par alimentation continue de chlorure d'hydrogène, l'addition de chlorure d'hydrogène, au sein du milieu réactionnel durant toute la durée de la réaction, soit sous forme gazeuse, soit sous forme liquide, soit sous la forme de tout composé autre que les réactifs qui peut générer du chlorure d'hydrogène dans le milieu réactionnel dans les conditions opératoires sélectionnées.

Dans un procédé d'hydrofluoration en continu, le rapport molaire entre le chlorure d'hydrogène ajouté par alimentation continue et l'hydrocarbure chloré introduit en continu au réacteur est généralement supérieur ou égal à 1. Avantageusement, ce rapport molaire est supérieur ou égal à 3. Ce rapport molaire est toutefois le plus souvent inférieur ou égal à 100. Avantageusement, ce rapport est inférieur ou égal à 50. D'une manière particulièrement préférée, ce rapport est supérieur ou égal à 5 et inférieur ou égal à 25.

Lorsque le procédé d'hydrofluoration selon l'invention se déroule de manière discontinue, l'alimentation continue en chlorure d'hydrogène peut être réalisée par exemple par introduction d'un courant de chlorure d'hydrogène gazeux au sein du milieu réactionnel durant toute la durée de la réaction. Dans ce cas, on règle le débit d'alimentation du chlorure d'hydrogène de telle sorte que le rapport entre la quantité totale de chlorure d'hydrogène introduite sur toute la durée de la réaction et la quantité d'hydrocarbure chloré initialement mise en oeuvre corresponde aux rapports molaires indiqués ci-dessus.

L'hydrocarbure chloré engagé dans le procédé d'hydrofluoration selon l'invention peut être un alcane aliphatique répondant à la formule générale C_{w}HₓCl_{y}F_{z} (I) dans laquelle w est un nombre entier compris entre 1 et 6, x est un nombre entier compris entre 0 et (2w + 1), y est un nombre entier compris entre 1 et (2w+1), z est un nombre entier compris entre 0 et (2w + 1) et la somme (x + y + z) a la valeur (2w + 2). Avantageusement, l'hydrocarbure chloré engagé dans le procédé selon l'invention est un alcane aliphatique répondant à la formule (I) dans laquelle w est un nombre entier compris entre 1 et 4, et x est un nombre entier compris entre 1 et 2w. A titre d'exemples non limitatifs d'alcanes chlorés engagés dans le procédé selon l'invention, on peut citer le dichlorométhane, le chlorofluorométhane, le chlorodifluorométhane, le 1-chloro-1-fluoroéthane, le 1,1-dichloro-1-fluoroéthane, le 1-chloro-1,1-difluoroéthane, les isomères du chlorotétrafluoroéthane, les isomères du dichlorotrifluoroéthane, les isomères du trichlorodifluoroéthane, les isomères du tétrachlorofluoroéthane, le pentachloroéthane, les composés de formule générale C₃H₃Cl_{(5-z)}F_{z}, C₄H₅Cl_{(5-z)}F_{z} avec z représentant un nombre entier pouvant prendre les valeurs de 0 à 4.

L'hydrocarbure chloré engagé dans le procédé d'hydrofluoration selon l'invention peut également être un alcène aliphatique répondant à la formule générale C_{w}HₓCl_{y}F_{z} (II) dans laquelle w est un nombre entier compris entre 1 et 6, x est un nombre entier compris entre 0 et (2w - 1), y est un nombre entier compris entre 1 et (2w - 1), z est un nombre entier compris entre 0 et (2w - 1) et la somme (x + y + z) a la valeur 2w. L'hydrocarbure chloré engagé dans le procédé selon l'invention peut également avantageusement être un alcène aliphatique répondant à la formule (I) dans laquelle w est un nombre entier compris entre 1 et 4. A titre d'exemples non limitatifs d'alcènes chlorés engagés dans le procédé selon l'invention, on peut citer le 1,1-dichloroéthylène, le trichloroéthylène, le perchloroéthylène, le chlorure de vinyle, le 3,3,3-trichloroprop-1-ène, le 1,1,3-trichloroprop-1-ène, le 1,1,3,3-tétrachlorobut-1-ène, le 1,1,1,3-tétrachlorobut-2-ène, le 1,1,1,3-tétrachlorobut-3-ène, le 1,1,4,4,4-pentachlorobut-1-ène, le 1,1,1,3-tétrachloroprop-2-ène, le 1,1,3,3-tétrachloroprop-1-ène, le 1,1,3,3-tétrachloro-2-méthylprop-2-ène, le 1,1,1,3-tétrachloro-2-méthylprop-2-ène, le 1-chloro-3,3,3-trifluoropropène ainsi que les mélanges de ces composés.

L'invention a donc pour but de produire, au départ d'hydrocarbures chlorés, saturés ou insaturés, des alcanes fluorés ou chlorofluorés qui contiennent plus d'atomes de fluor et moins d'atomes de chlore que les réactifs engagés. L'invention vise en particulier la synthèse d'hydrocarbures fluorés ou chlorofluorés tels que notamment le difluorométhane, le pentafluoroéthane, le 1,1,1,2-tétrafluoroéthane, le 1,1,1-trifluoroéthane, le 1,1-difluoroéthane, le 1,2,2-trichloro-1,1-difluoroéthane, le 2,2-dichloro-1,1,1-trifluoroéthane, le 1,1,1-trifluoro-2-chloroéthane, le 1,1,1,3-tétrafluoro-3-chloropropane, le 1,1,1,3,3-pentafluoropropane, le 1,1,1,3,3-pentafluorobutane, le 1,1,1,3,3,3-hexafluorobutane, le 1,1,1,3,3-pentafluoro-2-méthylpropane et le 1,1,1,3,3,3-hexafluoropropane, Le procédé d'hydrofluoration selon l'invention est particulièrement approprié à la préparation d'alcanes fluorés ne contenant pas d'atome de chlore dans leur structure moléculaire au départ d'hydrocarbures chlorés saturés, ainsi qu'à la préparation d'alcanes chlorofluorés au départ d'hydrocarbures chlorés insaturés.

Le procédé selon l'invention s'applique, par exemple, à la fabrication de 1,1,1,3,3-pentafluorapropane par réaction entre du fluorure d'hydrogène et un chloro(fluoro)propane de formule générale CCl₃₋ₐFₐ-CH₂-CHCl_{2-b}F_{b}, dans laquelle a est un nombre de 0 à 3 et b un nombre de 0 à 2.

Dans le procédé d'hydrofluoration selon la présente invention, le milieu réactionnel est avantageusement sous forme liquide et le catalyseur d'hydrofluoration qu'il contient est avantageusement choisi parmi les dérivés des métaux des groupes 3, 4, 5, 13, 14 et 15 du tableau périodique des éléments (IUPAC 1988) et leurs mélanges. On entend par dérivés des métaux, les hydroxydes, les oxydes et les sels inorganiques de ces métaux ainsi que leurs mélanges. On retient particulièrement les dérivés du titane, du niobium, du tantale, du molybdène, du bore, de l'étain et de l'antimoine. De préférence, le catalyseur est choisi parmi les dérivés des métaux des groupes 4, 5, 14 et 15 du tableau périodique des éléments, et plus particulièrement parmi les dérivés du titane, du tantale, de l'étain et de l'antimoine. Dans le procédé selon l'invention, les dérivés préférés des métaux sont leurs sels et ceux-ci sont de préférence choisis parmi les halogénures, et plus particulièrement parmi les chlorures, les fluorures et les chlorofluorures. Des catalyseurs d'hydrofluoration particulièrement avantageux dans le procédé selon l'invention sont les chlorures, les fluorures et les chlorofluorures de titane, d'étain et d'antimoine, notamment le tétrachlorure de titane, le tétrachlorure d'étain et le pentachlorure d'antimoine. Les catalyseurs comprenant un halogénure de titane sont préférés. Le tétrachlorure de titane seul ou en mélange avec d'autres catalyseurs est tout particulièrement préféré, notamment pour obtenir du 1,1,1,3,3-pentafluoropropane au départ de 1,1,1,3,3-pentachloropropane ou de 1-chloro-3,3,3-trifluoropropène.

Le rapport molaire entre le catalyseur et l'hydrocarbure chloré dans le milieu réactionnel est généralement supérieur ou égal à 0,001. De préférence, il est supérieur ou égal à 0,01. De très bons résultats ont été obtenus en présence d'au moins 0,1 mole de catalyseur par mole d'hydrocarbure chloré. En principe, il n'y a pas de limite supérieure à ce rapport. Il peut par exemple atteindre 1000. Généralement, il ne dépasse pas 100. Le plus souvent, il ne dépasse pas 10.

Le rapport molaire entre le catalyseur et le fluorure d'hydrogène dans le milieu réactionnel peut varier dans de larges limites. Il est généralement supérieur ou égal à 0.001. De préférence, il est supérieur ou égal à 0,01. De très bons résultats ont été obtenus en présence d'au moins 0,025 mole de catalyseur par mole de fluorure d'hydrogène. En général, ce rapport ne dépasse pas 10. Le plus souvent, il ne dépasse pas 1. De bons résultats ont été obtenus avec un rapport ne dépassant pas 0.75.

Le procédé d'hydrofluoration selon la présente invention peut être mis en oeuvre de manière continue ou discontinue. Il est entendu que les rapports molaires ci-dessus sont exprimés, dans le cas d'un procédé discontinu, par rapport aux quantités initiales d'hydrocarbure chloré et de fluorure d'hydrogène mises en oeuvre et. dans le cas d'un procédé continu, par rapport aux quantités stationnaires d'hydrocarbure chloré et de fluorure d'hydrogène dans le milieu réactionnel.

Dans le procédé d'hydrofluoration selon l'invention, on introduit dans le milieu réactionnel du fluorure d'hydrogène à l'état liquide ou gazeux et l'hydrocarbure chloré, de préférence à l'état liquide, dans un rapport molaire généralement supérieur ou égal à 5. De préférence, ce rapport molaire est supérieur ou égal à 10. Toutefois, ce rapport molaire est habituellement inférieur ou égal à 100. Avantageusement, ce rapport molaire est inférieur ou égal à 75, et d'une manière préférée, inférieur ou égal à 50.

Le procédé d'hydrofluoration selon l'invention peut être réalisé dans de larges gammes de températures et de pressions, de préférence choisies de manière à maintenir le milieu réactionnel sous forme liquide. Généralement, on travaille à une température d'au moins 75 °C. Une température d'au moins 90 °C est préférée. Une température d'au moins 100 °C est particulièrement préférée. Dans un procédé en phase liquide, en fonction notamment de la pression admissible, cette température ne dépasse le plus souvent pas 160 °C, les températures inférieures ou égales à 140 °C étant spécialement recommandées.

Généralement, on travaille à une pression d'au moins 2 bar. Une pression d'au moins 10 bar est préférée. Une pression d'au moins 15 bar est particulièrement préférée. Le plus souvent, cette pression ne dépasse pas 50 bar, les pressions inférieures ou égales à 30 bar étant spécialement recommandées.

Le procédé d'hydrofluoration selon l'invention peut être mis en oeuvre dans tout type de réacteur ou d'appareillage résistant à la pression, au fluorure d'hydrogène et au chlorure d'hydrogène et, dans le cas d'un procédé en continu, permettant de maintenir en permanence une composition sensiblement stable du milieu réactionnel. Le plus souvent, le procédé d'hydrofluoration selon l'invention est réalisé en continu dans un réacteur équipé d'un dispositif d'addition des réactifs, en phase liquide ou gazeuse, et de soutirage d'un courant de produits gazeux, par exemple dans un réacteur surmonté d'une colonne et d'un condenseur à reflux. Ce dispositif permet de maintenir en permanence une composition du milieu réactionnel conforme aux prescriptions exposées plus haut, par un réglage adéquat des conditions opératoires (notamment les débits des réactifs entrant dans le réacteur, la température et la pression dans le réacteur et la température dans le condenseur).

D'une façon surprenante. une alimentation continue de chlorure d'hydrogène dans le milieu réactionnel permet d'augmenter sensiblement la vitesse de conversion de l'hydrocarbure chloré, lorsque l'hydrocarbure chloré engagé est un alcane aliphatique de formule (I), d'augmenter la sélectivité en produit totalement fluoré et de diminuer l'accumulation de composés intermédiaires chlorofluorés, éventuellement insaturés, résultant d'un remplacement partiel des atomes de chlore des hydrocarbures chlorés et de l'élimination d'halogénure d'hydrogène (chlorure ou fluorure d'hydrogène) des réactifs ou des produits formés intermédiairement.

La présente invention concerne également un procédé pour la préparation du 1,1,1,3,3-pentafluoropropane au départ de 1,1,1,3,3-pentachloropropane comprenant deux étapes réactionnelles catalytiques, dans lequel on effectue de préférence une alimentation continue en chlorure d'hydrogène dans le milieu réactionnel d'au moins une des deux étapes réactionnelles. Ce procédé permet de produire le 1,1,1,3.3-pentafluoropropane (HFC-245fa) avec un rendement acceptable de manière industrielle au départ du 1,1,1,3,3-pentachloropropane (HCC-240fa).

Une première variante -- via le 1-chloro-3,3,3-trifluoropropène -- du procédé de préparation du 1,1,1,3,3-pentafluoropropane comprend :
- une première étape réactionnelle dans laquelle on fait réagir du 1,1,1,3,3-pentachloropropane (HCC-240fa) avec du fluorure d'hydrogène en phase liquide en présence d'un premier catalyseur d'hydrofluoration dans des conditions adéquates pour obtenir un mélange de produits réactionnels comprenant du 1-chloro-3,3,3-trifluoropropène (HCFC-1233zd) en quantité substantielle,
- une deuxième étape réactionnelle dans laquelle on fait réagir le 1-chloro-3,3,3-trifluoropropène (HCFC-1233zd) obtenu lors de la première étape avec du fluorure d'hydrogène en phase liquide en présence d'un deuxième catalyseur d'hydrofluoration et, de préférence, sous alimentation continue de chlorure d'hydrogène, pour obtenir du 1,1,1,3,3-pentafluoropropane (HFC-245fa).

Au cours ou au terme de la première étape, on procède avantageusement à un soutirage des produits volatils sous forme gazeuse. Par produits volatils, on entend le HCFC-1233zd, les hydrocarbures partiellement ou entièrement fluorés plus volatils que le HCFC-1233zd, le chlorure d'hydrogène co-produit, ainsi que le fluorure d'hydrogène n'ayant pas réagi.

Dans un premier mode de réalisation de cette première variante, on sépare le 1-chloro-3,3,3-trifluoropropène (HCFC-1233zd) et les autres hydrocarbures volatils partiellement ou entièrement fluorés obtenus des produits volatils soutirés pendant et/ou après la première étape et on les utilise - sans autre purification préalable - dans la deuxième étape pour les convertir en 1,1,1,3,3-pentafluoropropane (HFC-245fa). En d'autres termes, seuls le chlorure d'hydrogène et le fluorure d'hydrogène sont éliminés du mélange qui sera utilisé dans la deuxième étape.

Selon un autre mode de réalisation de cette première variante, qui est préféré, on utilise tous les produits volatils soutirés pendant et/ou après la première étape pour la préparation de 1,1,1,3,3-pentafluoropropane (HFC-245fa) dans la deuxième étape c.-à-d. que non seulement les produits partiellement ou entièrement fluorés dont le 1-chloro-3,3,3-trifluoropropène mais également le chlorure d'hydrogène et le fluorure d'hydrogène présents dans le milieu réactionnel de la première étape sont utilisés pour la préparation de 1,1,1,3,3-pentafluoropropane (HFC-245fa) dans la deuxième étape.

De manière similaire, au cours ou au terme de la deuxième étape, on procède avantageusement à un soutirage du HFC-245fa et des produits plus volatils que ce dernier sous forme gazeuse. Après séparation du HFC-245fa, les autres produits soutirés - principalement du chlorure d'hydrogène co-produit, du fluorure d'hydrogène ainsi qu'éventuellement certains hydrocarbures partiellement fluorés entraînés avec le HFC-245fa - peuvent être recyclés dans le procédé, de préférence au niveau de la deuxième étape réactionnelle.

D'une manière surprenante, il a en effet été découvert qu'une alimentation en continu de chlorure d'hydrogène dans un milieu réactionnel contenant du 1-chloro-3,3,3-trifluoropropène (HCFC-1233zd) comme hydrocarbure chloré permet d'augmenter sensiblement la vitesse de conversion du HCFC-1233zd en 1,1,1,3,3-pentafluoropropane (HFC-245fa), d'augmenter la sélectivité de la réaction et de diminuer l'accumulation de composés intermédiaires chlorofluorés. Il s'est avéré particulièrement avantageux d'envoyer à la deuxième étape le chlorure d'hydrogène co-produit lors de la première étape, et/ou de renvoyer à la deuxième étape une partie du chlorure d'hydrogène récupéré après la deuxième étape.

Dans cette première variante, on utilise de préférence deux catalyseurs d'hydrofluoration distincts. Des catalyseurs particulièrement adaptés à la préparation du 1-chloro-3,3,3-trifluoropropène (HCFC-1233zd) à partir de 1,1,1,3,3-pentachloropropane (HCC-240fa) et de fluorure d'hydrogène en phase liquide comprennent un ou plusieurs des éléments suivants : titane, étain, molybdène et/ou fer. Des catalyseurs particulièrement adaptés à la préparation du 1,1,1,3,3-pentafluoropropane (HFC-245fa) à partir de 1-chloro-3,3,3-trifluoropropène (HCFC-1233zd) et de fluorure d'hydrogène en phase liquide comprennent un ou plusieurs des éléments suivants : antimoine, thallium, niobium. Les catalyseurs sont avantageusement choisis parmi les dérivés des métaux susmentionnés et leurs mélanges. On entend par dérivés des métaux, les hydroxydes, les oxydes et les sels inorganiques de ces métaux ainsi que leurs mélanges. Les dérivés préférés des métaux sont leurs sels et ceux-ci sont de préférence choisis parmi les halogénures, et plus particulièrement parmi les chlorures, les fluorures et les chlorofluorures. Le tétrachlorure de titane, le tétrachlorure d'étain, le pentachlorure de molybdène et le trichlorure de fer ou bien un mélange d'au moins deux de ces produits, en particulier le tétrachlorure de titane seul ou en mélange avec d'autres catalyseurs, sont tout particulièrement préférés pour obtenir du 1-chloro-3,3,3-trifluoropropène au départ de 1,1,1,3,3-pentachloropropane. Le pentachlorure d'antimoine, le pentachlorure de thalium et le tetrachlorure de niobium ou bien un mélange d'au moins deux de ces produits, en particulier le pentachlorure d'antimoine seul ou en mélange avec d'autres catalyseurs, sont tout particulièrement préférés pour obtenir du 1,1,1,3,3-pentafluoropropane (HFC-245fa) au départ de 1-chloro-3,3,3-trifluoropropène (HCFC-1233zd).

Une deuxième variante -- via les dichlorotrifluoropropanes (HCFC-243) et le 1,1,1,3-tétrafluoro-3-chloropropane (HCFC-244fa) -- du procédé de préparation du 1,1,1,3,3-pentafluoropropane comprend :
- une première étape réactionnelle dans laquelle on fait réagir du 1,1,1,3,3-pentachloropropane (HCC-240fa) avec du fluorure d'hydrogène en phase liquide en présence d'un premier catalyseur d'hydrofluoration dans des conditions adéquates - notamment sous alimentation continue de chlorure d'hydrogène - pour obtenir un mélange de produits réactionnels constitué essentiellement (typiquement à plus de 80% en poids) de produits saturés, principalement des dichlorotrifluoropropanes (HCFC-243), du 1,1,1,3-tétrafluoro-3-chloropropane (HCFC-244fa) et du 1,1,1,3,3-pentafluoropropane (HFC-245fa),
- une deuxième étape réactionnelle dans laquelle on fait réagir les dichlorotrifluoropropanes (HCFC-243) et le 1,1,1,3-tétrafluoro-3-chloropropane (HCFC-244fa) obtenus lors de la première étape avec du fluorure d'hydrogène, en phase liquide ou en phase gazeuse, en présence d'un deuxième catalyseur d'hydrofluoration pour obtenir du 1,1,1,3,3-pentafluoropropane (HFC-245fa).

Au cours ou au terme de la première étape, on procède avantageusement à un soutirage des produits volatils sous forme gazeuse. Dans cette deuxième variante, par produits volatils, on entend essentiellement les dichlorotrifluoropropanes (HCFC-243), les chlorotétrafluoropropanes et le HFC-245fa déjà formé dans la première étape, le chlorure d'hydrogène co-produit, ainsi que le fluorure d'hydrogène n'ayant pas réagi.

Dans un premier mode de réalisation de cette deuxième variante, on sépare le mélange de dichlorotrifluoropropanes et de chlorotétrafluoropropanes des produits volatils soutirés pendant et/ou après la première étape et on les utilise - sans autre purification préalable - dans la deuxième étape pour les convertir en 1,1,1,3,3-pentafluoropropane (HFC-245fa). En d'autres termes, le chlorure d'hydrogène et le fluorure d'hydrogène sont éliminés du mélange qui sera utilisé dans la deuxième étape.

Selon un autre mode de réalisation de cette deuxième variante, on utilise tous les produits volatils soutirés pendant et/ou après la première étape pour la préparation de 1,1,1,3,3-pentafluoropropane (HFC-245fa) dans la deuxième étape c.-à-d. que non seulement les produits partiellement ou entièrement fluorés dont les dichlorotrifluoropropanes et chlorotétrafluoropropanes mais également le chlorure d'hydrogène et le fluorure d'hydrogène, ainsi qu'éventuellement le HFC-245fa déjà formé dans le milieu réactionnel de la première étape sont utilisés comme mélange de réactifs pour la préparation de 1,1,1,3,3-pentafluoropropane (HFC-245fa) dans la deuxième étape.

De manière similaire, au cours ou au terme de la deuxième étape, on procède avantageusement à un soutirage du HFC-245fa et des produits plus volatils que ce dernier sous forme gazeuse. Après séparation du HFC-245fa, les autres produits soutirés - principalement du chlorure d'hydrogène co-produit, du fluorure d'hydrogène ainsi qu'éventuellement certains hydrocarbures partiellement fluorés entraînés avec le HFC-245fa peuvent être recyclés dans le procédé, de préférence au niveau de la deuxième étape réactionnelle, sauf le chlorure d'hydrogène qui est de préférence renvoyé au niveau de la première étape réactionnelle.

D'une manière surprenante, il a en effet été découvert qu'une alimentation en continu de chlorure d'hydrogène dans un milieu réactionnel contenant du 1,1,1,3,3-pentachloropropane (HCC-240fa) comme hydrocarbure chloré permet d'augmenter sensiblement la vitesse de conversion du HCC-240fa, d'augmenter la sélectivité de la réaction en produits saturés fluorés, tels le 1,1,1-trifluoro-3,3-dichloropropane (HCFC-243fa), le 1,1,1,3-tétrafluoro-3-chloropropane (HCFC-244fa) et le 1,1,1,3,3-pentafluoropropane (HFC-245fa), de diminuer l'accumulation de composés insaturés peu réactifs dans le milieu réactionnel ainsi que de diminuer la formation de sous-produits lourds. Il s'est avéré dès lors particulièrement avantageux de renvoyer à la première étape tout ou partie du chlorure d'hydrogène co-produit lors de cette première étape et/ou de renvoyer à la première étape une partie du chlorure d'hydrogène récupéré après la deuxième étape.

Dans cette deuxième variante, on utilise de préférence deux catalyseurs d'hydrofluoration distincts. Des catalyseurs particulièrement adaptés à la préparation d'un mélange de produits réactionnels principalement saturés à partir de 1,1,1,3,3-pentachloropropane (HCC-240fa) et de fluorure d'hydrogène en phase liquide comprennent un ou plusieurs des éléments suivants : titane, étain, antimoine, niobium, et/ou tantale. Sous alimentation continue de chlorure d'hydrogène, les catalyseurs à base de titane conviennent particulièrement bien. Des catalyseurs particulièrement adaptés à la préparation du 1,1,1,3,3-pentafluoropropane (HFC-245fa) à partir des HCFC-243 et -244 et de fluorure d'hydrogène en phase liquide comprennent un ou plusieurs des éléments suivants : antimoine, thallium, niobium. Des catalyseurs particulièrement adaptés à la préparation du 1,1,1,3,3-pentafluoropropane (HFC-245fa) à partir des HCFC-243 et -244 et de fluorure d'hydrogène en phase gazeuse sont notamment les catalyseurs à base de chrome.

Les différentes étapes réactionnelles des deux variantes du procédé de préparation du 1,1,1,3,3-pentafluoropropane peuvent être réalisées dans des conditions opératoires classiquement utilisées. Pour les étapes en phase liquide, on peut typiquement travailler dans les conditions décrites plus haut dans le cadre du procédé d'hydrofluoration selon l'invention.

Les exemples ci-après illustrent l'invention de manière non limitative.

Dans les exemptes ci-dessous, le taux de transformation du 1,1,1,3,3-pentachloropropane est le rapport, exprimé en pourcent, entre la quantité mise en oeuvre diminuée de la quantité non convertie au terme de la réaction et la quantité mise en oeuvre: la sélectivité en 1,1,1,3,3-pentafluoropropane est le rapport entre la quantité de 1,1,1,3,3-pentafluoropropane formé et la quantité de 1,1,1,3,3-pentafluoropropane qui aurait été formée si tout le 1,1,1,3,3-pentachloropropane converti avait généré du 1,1,1,3,3-pentafluoropropane. Il en est de même pour les sélectivités en trifluorochloropropène (HCFC-1233), en dichlorotrifluoropropanes (HCFC-243) et en chlorotétratluoropropane (HCFC-244).

### Exemple 1

Dans un autoclave de 0,5 l en acier inoxydable, équipé d'un agitateur mécanique à pales, d'une sonde de température et d'un tube plongeant permettant d'effectuer des prélèvements en phase liquide en cours d'essai, on a introduit 0.23 mole de 1,1,1,3,3-pentachloropropane, 0.04 mole de tétrachlorure de titane et 9 moles de fluorure d'hydrogène. L'autoclave a ensuite été plongé dans un bain thermostatisé, maintenu à une température de 120 °C sous agitation continue pendant 22 heures. Du chlorure d'hydrogène gazeux a été introduit en continu, dans l'autoclave, à un débit de 0,2 mole/heure. La pression a été régulée à 25 bar. Un prélèvement réalisé après 2,5 heures de réaction a montré que le taux de transformation du 1,1,1,3,3-pentachloropropane mis en oeuvre était supérieur à 99 % molaire avec une sélectivité en HCFC-243 de 20,6 %, en HCFC-244 de 62.2 %, en HCFC-1233 de 3,3 % et en 1,1,1,3,3-pentafluoropropane de 11 %. Après 22 heures de réaction, la sélectivité en HCFC-243 était de 13,9 %, celle en HCFC-244 était de 41,9 %, celle en HCFC-1233 était de 1,5 % et celle en 1,1,1,3,3-pentafluoropropane était de 41,5 %.

### Exemple 2

L'essai de l'exemple 1 a été répété mais sans alimentation continue de chlorure d'hydrogène. Un prélèvement réalisé après 2,5 heures de réaction a montré que le taux de transformation du 1,1,1,3,3-pentachloropropane mis en oeuvre était de 77 % molaire avec une sélectivité en HCFC-243 de 0,1 %, en HCFC-244 de 8,7 %, en HCFC-1233 de 64,6 % et en 1,1,1,3,3-pentafluoropropane de 0,7 %. Après 22 heures de réaction. le taux de transformation du 1,1,1,3,3-pentachloropropane était de 99 % et la sélectivité en HCFC-243 était de 8.2 %, celle en HCFC-244 était de 73,9 %, celle en HCFC-1233 était de 11,9 % et celle en 1,1,1,3,3-pentafluoropropane était seulement de 4,3 %.

## Revendications

1. Procédé d'hydrofluoration d'un hydrocarbure chloré par réaction avec du fluorure d'hydrogène dans un milieu réactionnel comprenant un catalyseur d'hydrofluoration, **caractérisé par** une alimentation continue de chlorure d'hydrogène dans le milieu réactionnel.

2. Procédé selon la revendication 1, mené en continu, dans lequel le rapport molaire entre le chlorure d'hydrogène ajouté par alimentation continue et l'hydrocarbure chloré introduit est supérieur ou égal à 1 et inférieur ou égal à 100.

3. Procédé selon la revendication 1, mené en discontinu, dans lequel l'alimentation en chlorure d'hydrogène dans le milieu réactionnel est réglée de telle sorte que le rapport entre la quantité totale de chlorure d'hydrogène introduite sur toute la durée de la réaction et la quantité d'hydrocarbure chloré initialement mise en oeuvre est supérieur ou égal à 1 et inférieur ou égal à 100.

4. Procédé selon l'une quelconque des revendications 1 à 3 dans lequel l'hydrocarbure chloré est un alcane aliphatique répondant à la formule générale C_{w}HₓCl_{y}F_{z} (I) dans laquelle w est un nombre entier compris entre 1 et 6, x est un nombre entier compris entre 0 et (2w + 1), y est un nombre entier compris entre 1 et (2w + 1), z est un nombre entier compris entre 0 et (2w + 1) et la somme (x + y + z) a la valeur (2w + 2).

5. Procédé selon l'une quelconque des revendications 1 à 3 dans lequel l'hydrocarbure chloré est un alcène aliphatique répondant à la formule générale C_{w}HₓCl_{y}F_{z} (I) dans laquelle w est un nombre entier compris entre 1 et 6, x est un nombre entier compris entre 0 et (2w - 1), y est un nombre entier compris entre 1 et (2w - 1), z est un nombre entier compris, entre 0 et (2w - 1) et la somme (x + y + z) a la valeur 2w.

6. Procédé selon l'une quelconque des revendications 1 à 5 **caractérisé en ce qu'**il se déroule en phase liquide.

7. Procédé selon l'une quelconque des revendications 1 à 6 dans lequel on réalise la réaction à une température de 75 à 160 °C et à une pression de 2 à 50 bar.

8. Procédé selon l'une quelconque des revendications 1 à 7 dans lequel le rapport molaire entre le catalyseur et l'hydrocarbure chloré dans le milieu réactionnel est de 0,001 à 1000 et dans lequel le rapport molaire entre le catalyseur et le fluorure d'hydrogène dans le milieu réactionnel est de 0,001 à 10.

9. Procédé d'hydrofluoration selon l'une quelconque des revendications 1 à 8, appliqué à la fabrication de 1,1,1,3,3-pentafluoropropane par réaction entre du fluorure d'hydrogène et un chloro(fluoro)propane de formule générale CCl₃₋ₐFₐ-CH₂-CHCl_{2-b}F_{b}, dans laquelle a est un nombre de 0 à 3 et b un nombre de 0 à 2.

10. Procédé d'hydrofluoration selon l'une quelconque des revendications 1 à 8, appliqué à la fabrication de 1,1,1,3,3-pentafluoropropane par réaction entre du fluorure d'hydrogène et du 1-chloro-3,3,3-trifluoropropène.

11. Procédé d'hydrofluoration selon l'une quelconque des revendications 1 à 8, appliqué à la fabrication d'un mélange de produits constitué essentiellement de produits saturés, principalement des dichlorotrifluoropropanes (HCFC-243), du 1,1,1,3-tétrafluoro-3-chloropropane (HCFC-244fa) et du 1,1,1,3,3-pentafluoropropane (HFC-245fa), par réaction entre du fluorure d'hydrogène et du 1,1,1,3,3-pentachloropropane.

12. Procédé selon l'une quelconque des revendications 1 à 8, appliqué à la préparation du 1,1,1,3,3-pentafluoropropane au départ de 1,1,1,3,3-pentachloropropane et comprenant deux étapes réactionnelles catalytiques, procédé dans lequel on effectue une alimentation continue en chlorure d'hydrogène dans le milieu réactionnel d'au moins une des deux étapes réactionnelles.

13. Procédé selon la revendication 12 comprenant
- une première étape réactionnelle dans laquelle on fait réagir du 1,1,1,3,3-pentachloropropane (HCC-240fa) avec du fluorure d'hydrogène en phase liquide en présence d'un premier catalyseur d'hydrofluoration pour obtenir un mélange de produits réactionnels comprenant du 1-chloro-3,3,3-trifluoropropène (HCFC-1233zd) en quantité substantielle,
- une deuxième étape réactionnelle dans laquelle on fait réagir le 1-chloro-3,3,3-trifluoropropène (HCFC-1233zd) obtenu lors de la première étape avec du fluorure d'hydrogène en phase liquide en présence d'un deuxième catalyseur d'hydrofluoration et sous alimentation continue de chlorure d'hydrogène, pour obtenir du 1,1,1,3,3-pentafluoropropane (HFC-245fa).

14. Procédé selon la revendication 13, dans lequel on procède, au cours ou au terme de la première étape, à un soutirage sous forme gazeuse du chloro-3,3,3-trifluoropropène (HCFC-1233zd) et des produits plus volatils que le HCFC-1233zd.

15. Procédé selon la revendication 14, dans lequel on utilise tous les produits volatils soutirés pendant et/ou après la première étape pour la préparation de 1,1,1,3,3-pentafluoropropane (HFC-245fa) dans la deuxième étape.

16. Procédé selon une quelconque des revendications 13 à 15, dans lequel on introduit en continu du chlorure d'hydrogène dans le milieu réactionnel de la deuxième étape.

17. Procédé de fabrication du 1,1,1,3,3-pentafluoropropane (EFC-245fa) selon la revendication 12 comprenant
- une première étape réactionnelle dans laquelle on fait réagir du 1,1,1,3,3-pentachloropropane (HCC-240fa) avec du fluorure d'hydrogène en phase liquide en présence d'un premier catalyseur d'hydrofluoration, sous alimentation continue de chlorure d'hydrogène, pour obtenir un mélange de produits réactionnels constitué essentiellement de produits saturés, principalement des dichlorotrifluoropropanes (HCFC-243), du 1,1,1,3-tétrafluoto-3-chloropropane (HCFC-244fa) et du 1,1,1,3,3-pentafluoropropane (HFC-245fa),
- une deuxième étape réactionnelle dans laquelle on fait réagir les dichlorotrifluoropropanes (HCFC-243) et le 1,1,1,3-tétrafluoro-3-chloropropane (HCFC-244fa) obtenus lors de la première étape avec du fluorure d'hydrogène, en phase liquide ou en phase gazeuse, en présence d'un deuxième catalyseur d'hydrofluoration pour obtenir du 1,1,1,3,3-pentafluoropropane (HFC-245fa).

18. Procédé selon la revendication 17, dans lequel on procède, au cours ou au terme de la première étape, à un soutirage sous forme gazeuse des dichlorotrifluoropropanes (HCFC-243) et des produits plus volatils que les dichlorotrifluoropropanes.

19. Procédé selon la revendication 17 ou 18, dans lequel on introduit en continu du chlorure d'hydrogène dans le milieu réactionnel de la première étape.

20. Procédé selon une quelconque des revendications 17 à 19, dans lequel on réalise la première étape en présence d'un catalyseur à base de titane.

## Claims

1. Process for the hydrofluorination of a chlorohydrocarbon by reaction with hydrogen fluoride in a reaction medium comprising a hydrofluorination catalyst **characterized by** a continuous feed of hydrogen chloride into the reaction medium.

2. Process as claimed in claim 1, carried out in continuous mode, in which the molar ratio between the hydrogen chloride added by continuous feed and the chlorohydrocarbon introduced is greater than or equal to 1 and less than or equal to 100.

3. Process as claimed in claim 1, carried out in batchwise mode, in which the hydrogen chloride fed into the reaction medium is adjusted such that the ratio between the total amount of hydrogen chloride introduced throughout the reaction and the amount of chlorohydrocarbon initially used is greater than or equal to 1 and less than or equal to 100.

4. Process as claimed in any one of claims 1 to 3, in which the chlorohydrocarbon is an aliphatic alkane corresponding to the general formula C_{w}HₓCl_{y}F_{z} (I) in which w is an integer between 1 and 6, x is an integer between 0 and (2w+1), y is an integer between 1 and (2w+1), z is an integer between 0 and (2w+1) and the sum (x+y+z) is equal to (2w+2).

5. Process as claimed in any one of claims 1 to 3, in which the chlorohydrocarbon is an aliphatic alkene corresponding to the general formula C_{w}HₓCl_{y}F_{z} (I) in which w is an integer between 1 and 6, x is an integer between 0 and (2w-1), y is an integer between 1 and (2w-1), z is an integer between 0 and (2w-1) and the sum (x+y+z) is equal to 2w.

6. Process as claimed in any one of claims 1 to 5, **characterized in that** it is carried out in liquid phase.

7. Process as claimed in any one of claims 1 to 6, in which the reaction is carried out at a temperature of about 75 to 160°C, at a pressure of about 2 to 50 bar.

8. Process as claimed in any one of claims 1 to 7, in which the molar ratio between the catalyst and the chlorohydrocarbon in the reaction medium is from 0.001 to 1000 and in which the molar ratio between the catalyst and the hydrogen fluoride in the reaction medium is from 0.001 to 10.

9. Hydrofluorination process as claimed in any one of claims 1 to 8, applied to the manufacture of 1,1,1,3,3-pentafluoropropane by reaction between hydrogen fluoride and a chloro(fluoro)propane of general formula CCl₃₋ₐFₐ-CH₂-CHCl_{2-b}F_{b}, in which a is a number from 0 to 3 and b is a number from 0 to 2.

10. Hydrofluorination process as claimed in any one of claims 1 to 8, applied to the manufacture of 1,1,1,3,3-pentafluoropropane by reaction between hydrogen fluoride and 1-chloro-3,3,3-trifluoropropene.

11. Hydrofluorination process as claimed in any one of claims 1 to 8, applied to the manufacture of a mixture of products consisting essentially of saturated products, mainly dichlorotrifluoropropanes (HCFC-243), 1,1,1,3-tetrafluoro-3-chloropropane (HCFC-244fa) and 1,1,1,3,3-pentafluoropropane (HFC-245fa), by reaction between hydrogen fluoride and 1,1,1,3,3-pentachloropropane.

12. Process according to anyone of Claims 1 to 8, applied to preparation of 1,1,1,3,3-pentafluoropropane starting with 1,1,1,3,3-pentachloropropane and comprising two catalytic reaction steps, in which process hydrogen chloride is fed continuously into the reaction medium of at least one of the two reaction steps.

13. Process according to Claim 12 comprising
- a first reaction step in which 1,1,1,3,3-penta-chloropropane (HCC-240fa) is reacted with hydrogen fluoride in liquid phase in the presence of a first hydrofluorination catalyst to obtain a mixture of reaction products comprising 1-chloro-3,3,3-trifluoropropene (HCFC-1233zd) in substantial amount,
- a second reaction step in which the 1-chloro-3,3,3-trifluoropropene (HCFC-1233zd) obtained from the first step is reacted with hydrogen fluoride in liquid phase in the presence of a second hydrofluorination catalyst, and while hydrogen chloride is continuously fed in, in orderto obtain 1,1,1,3,3-pentafluoropropane (HFC-245fa).

14. Process as claimed in claim 13, in which chloro-3,3,3-trifluoropropene (HCFC-1233zd) and products more volatile than HCFC-1233zd are removed in gaseous form during or at the end of the first step.

15. Process as claimed in claim 14, in which all the volatile products removed during and/or after the first step are used for the preparation of 1,1,1,3,3-pentafluoropropane (HFC-245fa) in the second step.

16. Process as claimed in any one of claims 13 to 15, in which hydrogen chloride is introduced continuously into the reaction medium of the second step.

17. Process for manufacturing 1,1,1,3,3-pentafluoropropane (HFC-245fa) according to Claim 12 comprising
- a first reaction step in which 1,1,1,3,3-penta-chloropropane (HCC-240fa) is reacted with hydrogen fluoride in liquid phase in the presence of a first hydrofluorination catalyst with a continuous feed of hydrogen chloride in order to obtain a mixture of reaction products consisting essentially of saturated products, mainly dichlorotrifluoropropanes (HCFC-243), 1,1,1,3-tetrafluoro-3-chloropropane (HCFC-244fa) and 1,1,1,3,3-pentafluoropropane (HFC-245fa),
- a second reaction step in which the dichloro-trifluoropropanes (HCFC-243) and 1,1,1,3-tetrafluoro-3-chloropropane (HCFC-244fa) obtained during the first step are reacted with hydrogen fluoride, in liquid phase or in gas phase, in the presence of a second hydrofluorination catalyst in order to obtain 1,1,1,3,3-pentafluoropropane (HFC-245fa).

18. Process as claimed in claim 17, in which dichlorotrifluoropropanes (HCFC-243) and products more volatile than the dichlorotrifluoropropanes are removed in gaseous form during or at the end of the first step.

19. Process as claimed in claim 17 or 18, in which hydrogen chloride is introduced continuously into the reaction medium of the first step.

20. Process as claimed in any one of claims 17 to 19, in which the first step is carried out in the presence of a titanium-based catalyst.

## Patentansprüche

1. Verfahren zur Hydrofluorierung eines chlorierten Kohlenwasserstoffes durch Umsetzung mit Fluorwasserstoff in einem einen Hydrofluorierungskatalysator umfassenden Reaktionsmilieu, **gekennzeichnet durch** eine kontinuierliche Einspeisung von Chlorwasserstoff in das Reaktionsmilieu.

2. Verfahren nach Anspruch 1, das kontinuierlich ausgeführt wird, worin das Molverhältnis zwischen dem durch kontinuierliche Einspeisung zugesetzten Chlorwasserstoff und dem eingebrachten chlorierten Kohlenwasserstoff größer oder gleich 1 und kleiner oder gleich 100 ist.

3. Verfahren nach Anspruch 1, das diskontinuierlich ausgeführt wird, worin die Einspeisung des Chlor-wasserstoffes in das Reaktionsmilieu derart geregelt wird, daß das Verhältnis zwischen der über die gesamte Reaktionsdauer eingeführten Gesamtmenge an Chlorwasserstoff zu der ursprünglich eingesetzten Menge an chloriertem Kohlenwasserstoff größer oder gleich 1 und kleiner oder gleich 100 ist.

4. Verfahren nach einem der Ansprüche 1 bis 3, worin der chlorierte Kohlenwasserstoff ein aliphatisches Alkan entsprechend der allgemeinen Formel C_{w}HₓCl_{y}F_{z} (I) ist, worin w eine ganze Zahl zwischen 1 und 6 darstellt, x eine ganze Zahl zwischen 0 und (2w + 1) ist, y eine ganze Zahl zwischen 1 und (2w + 1) darstellt, z eine ganze Zahl zwischen 0 und (2w + 1) ist und die Summe (x + y + z) den Wert (2w + 2) aufweist.

5. Verfahren nach einem der Ansprüche 1 bis 3, worin der chlorierte Kohlenwasserstoff ein aliphatisches Alken entsprechend der allgemeinen Formel C_{w}HₓCl_{y}F_{z} (I) ist, worin w eine ganze Zahl zwischen 1 und 6 darstellt, x eine ganze Zahl zwischen 0 und (2w - 1) ist, y eine ganze Zahl zwischen 1 und (2w - 1) darstellt, z eine ganze Zahl zwischen 0 und (2w - 1) ist und die Summe (x + y + z) den Wert 2w aufweist.

6. Verfahren nach einem der Ansprüche 1 bis 5, **dadurch gekennzeichnet, daß** es in flüssiger Phase abläuft.

7. Verfahren nach einem der Ansprüche 1 bis 6, worin die Umsetzung bei einer Temperatur von 75 bis 160°C und bei einem Druck von 2 bis 50 bar ausgeführt wird.

8. Verfahren nach einem der Ansprüche 1 bis 7, worin das Molverhältnis zwischen dem Katalysator und dem chlorierten Kohlenwasserstoff im Reaktionsmilieu von 0,001 bis 1.000 beträgt und worin das Molverhältnis zwischen dem Katalysator und dem Fluorwasserstoff im Reaktionsmilieu von 0,001 bis 10 beträgt.

9. Hydrofluorierungsverfahren nach einem der Ansprüche 1 bis 8, angewendet auf die Herstellung von 1,1,1,3,3-Pentafluorpropan durch Reaktion zwischen Fluorwasserstoffund einem Chlor(fluor)propan der allgemeinen Formel CCl₃₋ₐFₐ-CH₂CHCl_{2-b}F_{b}, worin a eine Zahl von 0 bis 3 und b eine Zahl von 0 bis 2 bedeuten.

10. Hydrofluorierungsverfahren nach einem der Ansprüche 1 bis 8, angewendet auf die Herstellung von 1,1,1,3,3-Pentafluorpropan durch Reaktion zwischen Fluorwasserstoff und 1-Chlor-3,3,3-trifluorpropen.

11. Hydrofluorierungsverfahren nach einem der Ansprüche 1 bis 8, angewendet auf die Ausbildung eines Gemisches von Produkten, das im wesentlichen aus gesättigten Produkten gebildet ist, vorwiegend aus Dichlortrifluorpropanen (HCFC-243), 1,1,1,3-Tetrafluor-3-chlorpropan (HCFC-244fa) und aus 1,1,1,3,3-Pentafluorpropan (HFC-245fa), durch Umsetzung zwischen Fluorwasserstoff und 1,1,1,3,3-Pentachlorpropan.

12. Verfahren nach einem der Ansprüche 1 bis 8, angewendet auf die Herstellung von 1,1,1,3,3-Pentafluorpropan aus 1,1,1,3,3-Pentachlorpropan und umfassend zwei katalytische Reaktionsstufen, in welchem Verfahren eine kontinuierliche Einspeisung von Chlorwasserstoff in das Reaktionsmilieu wenigstens einer der beiden Reaktionsstufen vorgenommen wird.

13. Verfahren nach Anspruch 12, umfassend
- eine erste Reaktionsstufe, worin 1,1,1,3,3-Pentachlorpropan (HCC-240fa) mit Fluorwasserstoff in flüssiger Phase in Gegenwart eines ersten Hydrofluorierungskatalysators umgesetzt wird, um ein Gemisch von Reaktionsprodukten zu erhalten, das 1-Chlor-3,3,3-trifluorpropen (HCFC-1233zd) in substantieller Menge enthält,
- eine zweite Reaktionsstufe, worin das in der ersten Stufe erhaltene 1-Chlor-3,3,3-trifluorpropen (HCFC-1233zd) in flüssiger Phase in Gegenwart eines zweiten Hydrofluorierungskatalysators mit Fluorwasserstoffund unter kontinuierlicher Einspeisung von Chlorwasserstoff umgesetzt wird, um 1,1,1,3,3-Pentafluorpropan (HFC-245fa) zu erhalten.

14. Verfahren nach Anspruch 13, worin während der oder am Ende der ersten Stufe Chlor-3,3,3-trifluorpropen (HCFC-1233zd) und flüchtigere Produkte als das HCFC-1233zd in Gasform abgezogen werden.

15. Verfahren nach Anspruch 14, worin alle während und/oder nach der ersten Stufe abgezogenen flüchtigen Produkte zur Herstellung von 1,1,1,3,3-Pentafluorpropan (HFC-245fa) in der zweiten Stufe verwendet werden.

16. Verfahren nach einem der Ansprüche 13 bis 15, worin Chlorwasserstoff kontinuierlich in das Reaktionsmilieu der zweiten Stufe eingeführt wird.

17. Verfahren zur Herstellung von 1,1,1,3,3-Pentafluorpropan (HFC-245fa) nach Anspruch 12, umfassend
- eine erste Reaktionsstufe, worin 1,1,1,3,3-Pentachlorpropan (HCC-240fa) mit Fluorwasserstoff in flüssiger Phase in Gegenwart eines ersten Hydrofluorierungskatalysators unter kontinuierlicher Einspeisung von Chlorwasserstoffumgesetzt wird, um ein Gemisch von Reaktionsprodukten zu erhalten, das im wesentlichen aus gesättigten Produkten besteht, vorwiegend aus Dichlortrifluorpropanen (HCFC-243), 1,1,1,3,3-Tetrafluor-3-chlorpropan (HCFC-244fa) und aus 1,1,1,3,3-Pentafluorpropan (HFC-245fa),
- eine zweite Reaktionsstufe, worin die in der ersten Stufe erhaltenen Dichlortrifluorpropane (HCFC-243) und das 1,1,1,3-Tetrafluor-3-chlorpropan (HCFC-244fa) mit Fluorwasserstoff in flüssiger Phase oder in der Gasphase in Gegenwart eines zweiten Hydrofluorierungskatalysators umgesetzt werden, um das 1, 1, 1,3,3-Pentafluorpropan (HFC-245fa) zu erhalten.

18. Verfahren nach Anspruch 17, worin während oder am Ende der ersten Stufe die Dichlortrifluorpropane (HCFC-243) und die flüchtigeren Produkte als die Dichlortrifluorpropane in Gasform abgezogen werden.

19. Verfahren nach Anspruch 17 oder 18, worin Chlorwasserstoff kontinuierlich in das Reaktionsmilieu der ersten Stufe eingeführt wird.

20. Verfahren nach einem der Ansprüche 17 bis 19, worin die erste Stufe in Gegenwart eines Katalysators auf Titanbasis ausgeführt wird.
